# FASCICULE DE BREVET EUROPEEN

(11) **EP 1 479 683 B1**
(45) Date de publication et mention de la délivrance du brevet: **26.10.2005**
(21) Numéro de dépôt: 04291080.2
(22) Date de dépôt: 27.04.2004
(51) Int. Cl.: C07D 487/22, C06B 25/34

(54) **Procédé de synthèse de l'hexanitrohexaazaisowurtzitane en 2 étapes à partir d'un amine primaire**
Prozess zur Herstellung von Hexanitrohexaazaisowurtzitan in zwei Stufen ausgehend von einem primären Amin
Process for the synthesis of hexanitrohexaazaisowurtzitane in 2 steps from a primary amine

(30) Priorité: 22.05.2003 FR 0306160
(43) Date de publication de la demande: 24.11.2004
(73) Titulaire: SNPE Matériaux Energétiques, 75004 Paris (FR)
(72) Inventeur: Cagnon, Guy, 91610 Ballancourt (FR); Eck, Geneviève, 84170 Monteux (FR); Herve, Grégoire, 91610 Ballancourt (FR); Jacob, Guy, 91710 Vert Le Petit (FR)
(74) Mandataire: Le Roux, Martine

(56) Documents cités:
- WO-A-00/52011
- US-A- 5 693 794
- US-A- 6 147 209

## Description

La présente invention se situe dans le domaine des poudres pour armes, propergols et explosifs, très couramment utilisés, notamment dans les industries d'armement.

Elle a plus précisément pour objet un nouveau procédé de synthèse du 2,4,6,8,10,12-hexanitro-2,4,6,8,10,12-hexaazatétracyclo (5.5.0.0^{5,9}.0^{3,11}) dodécane, couramment appelé hexanitrohexaazaisowurtzitane ou HNIW.

Il est connu d'utiliser l'HNIW comme charge énergétique dans les poudres, propergols et explosifs, notamment en remplacement de tout ou partie de l'octogène et de l'hexogène. L'HNIW constitue actuellement la charge énergétique la plus performante du fait de sa densité élevée et de sa forte enthalpie de formation, mais le développement de l'HNIW pour remplacer l'octogène et/ou l'hexogène n'est envisageable que si son coût d'obtention est réduit de façon importante.

En effet, les procédés de synthèse actuellement connus de l'HNIW sont tous très coûteux.

Le brevet US 5.693.794 décrit par exemple la synthèse de l'HNIW en 4 étapes à partir de benzylamine et de glyoxal.

Dans une première étape, par réaction de la benzylamine avec le glyoxal, on obtient le 2,4,6,8,10,12-hexabenzyl-2,4,6,8,10,12-hexaazatétracyclo (5.5.0.0^{5,9}.0^{3,11}) dodécane, couramment appelé hexabenzylhexaazaisowurtzitane ou HBIW.

Dans une seconde étape, on hydrogénolyse l'HBIW dans l'anhydride acétique à chaud en présence d'un catalyseur, ce qui permet d'obtenir le 2,6,8,12-tétraacétyl-4,10-dibenzyl-2,4,6,8,10,12-hexaazaisowurtzitane, couramment appelé tétraacétyldibenzylhexaazaisowurtzitane ou TADBIW. Le catalyseur est très coûteux et sa filtration et son élimination sont difficiles.

Dans une troisième étape, on remplace les 2 groupements benzyles du TADBIW par des groupements nitroso pour former le tétraacétyldinitrosohexaazaisowurtzitane (TADNIW) par réaction du TADBIW avec le tétrafluoroborate de nitrosonium, réactif coûteux.

Dans une quatrième étape, on remplace les 4 groupements acétyles et les 2 groupements nitroso du TADNIW pour former HNIW par réaction de nitrolyse du TADNIW par le tétrafluoroborate de nitronium, réactif coûteux.

Cette synthèse de l'HNIW en 4 étapes à partir de la benzylamine et du glyoxal est donc particulièrement coûteuse, notamment du fait du nombre important d'étapes, de temps de réaction longs et du prix élevé des réactifs.

Des améliorations de ce procédé sont connues, et consistent principalement à supprimer la troisième étape précitée en remplaçant le TADBIW par un intermédiaire directement nitrable, tel que le tétraacétylisowurtzitane (TAIW), le tétraacétylmonoformylisowurtzitane (TAMFIW) ou l'hexaacétylisowurtzitane (HAIW), obtenus en poussant plus loin l'hydrogénolyse lors de la seconde étape. Cette réaction d'hydrogénolyse poussée de l'HBIW doit être complète pour ne pas conserver de groupements benzyles qui ne sont pas directement nitrolysables. Cette contrainte impose d'augmenter le taux de catalyseur servant à l'hydrogénolyse et amène le coût du TAIW, du TAMFIW ou de l'HAIW à une valeur supérieure à celle du TADBIW, ce qui limite l'avantage résultant de la suppression de la troisième étape.

Il est également connu, lors de la dernière étape de nitration, de remplacer le tétrafluoroborate de nitronium par un mélange sulfonitrique moins coûteux.

Les brevets US 6.147.209, EP 753519 et WO 00/52011 décrivent par exemple de telles améliorations.

Malgré ces améliorations, le coût d'obtention de l'HNIW reste très élevé, essentiellement à cause des 2 étapes incontournables de synthèse de l'intermédiaire HBIW, puis de son hydrogénolyse en présence d'un catalyseur coûteux. C'est pourquoi, depuis très longtemps, l'homme du métier cherche une nouvelle voie de synthèse de l'HNIW, qui ne serait pas une simple amélioration des procédés existants, mais qui serait une voie différente, simple, nettement moins coûteuse, ne faisant pas appel à l'HBIW comme composé intermédiaire et ne comprenant pas d'étape coûteuse d'hydrogénolyse catalytique.

Aucune solution à ce problème, n'est, à notre connaissance, décrite dans l'état de la technique.

La présente invention propose une telle solution.

Elle a pour objet un nouveau procédé de synthèse de l'hexanitrohexaazaisowurtzitane comprenant une première étape de réaction d'un dérivé α,β-dicarbonylé, de préférence le glyoxal, avec une amine primaire, permettant de former un dérivé 2,4,6,8,10,12-hexasubstitué-2,4,6,8,10,12-hexaazatétracyclo(5.5.0.0^{5,9}.0^{3,11})dodécane, appelé par la suite plus brièvement dérivé hexasubstitué de l'hexaazaisowurtzitane.

Ce nouveau procédé est caractérisé en ce que l'hexanitrohexaazaisowurtzitane est directement obtenu, en une seule étape réactionnelle, par nitration du dérivé hexasubstitué de l'hexaazaisowurtzitane formé par réaction du dérivé α,β-dicarbonylé avec l'amine primaire.

Ce procédé est particulièrement simple et très peu coûteux. Il ne nécessite que 2 étapes réactionnelles pour obtenir l'HNIW à partir du glyoxal et d'une amine primaire, sans étape d'hydrogénolyse.

Ce procédé est également particulièrement inattendu.

En effet, selon la présente invention, la cyclisation d'un dérivé α,β-dicarbonylé avec une amine primaire judicieusement choisie forme une cage hexaazaisowurtzitane dont les 6 atomes sont substitués par des groupements directement nitrolysables, ce qui n'est pas le cas des groupements benzyles dans HBIW.

Or, tous les essais décrits dans l'état de la technique pour obtenir une cage hexaazaisowurtzitane à partir de glyoxal et d'une amine primaire autre que la benzylamine, ou d'une benzylamine dont le cycle phényle est substitué, se sont révélés infructueux.

NIELSEN et coll., dans l'article "Polyazapolycyclics by condensation of aldehydes with amines. Formation of 2,4,6,8,10,12-hexabenzyl-2,4,6,8,10,12-hexaazatétracyclo(5.5.0.0^{5,9}.0^{3,11})dodécanes from glyoxal and benzylamines", J. Org. Chem. 1990,55, 1459-1466, mentionnent par exemple que la réaction des amines avec le glyoxal pour former des dérivés hexaazaisowurtzitanes apparaît être limitée à la benzylamine et à certaines benzylamines dont le cycle phényle est substitué, et que tous les essais effectués à partir notamment d'hétéroarylméthylamines et d'allylamines ont été infructueux.

SURAPANENI et DAMAVARAPU, lors de la 31^{ème} conférence annuelle internationale de l'ICT (Institut Chemische Technologie), 27-30 juin 2000 à Karlsruhe (Allemagne), Energetic Materials, Analysis, Diagnostics and Testing, Process improvements in CL-20 manufacture, pages 108-1 à 108-4, mentionnent d'une part, les inconvénients de la nécessité d'utiliser la benzylamine comme amine de départ, et d'autre part, que seules les amines benzyliques permettent d'obtenir le squelette isowurtzitane, tous les essais réalisés avec d'autres amines ou amides ayant échoués.

La présente invention a donc vaincu un préjugé très tenace, établi depuis de nombreuses années, puis plusieurs fois confirmé.

Selon la présente invention, si l'on représente l'amine primaire de départ par la formule générale R-NH2, R étant un groupement organique, la cyclisation de cette amine primaire avec un dérivé α,β-dicarbonylé forme un dérivé 2,4,6,8,10,12-hexaR-2,4,6,8,10,12-hexaazatétracyclo (5.5.0.0^{5,9}.0^{3,11})dodécane, c'est-à-dire un dérivé hexasubstitué de l'hexaazaisowurtzitane dont les 6 substituants sont des groupements R, et dont les 6 liaisons N-R sont directement nitrolysables, c'est-à-dire dont les 6 groupements R sont suffisamment électrophiles pour être déplacés par un ion nitronium lorsque celui-ci attaque le doublet libre des atomes d'azote, en formant un cation R⁺ suffisamment stabilisé au cours de cette substitution.

De façon préférée, selon la présente invention, le groupement organique R est choisi dans le groupe constitué par les groupements hétéroarylméthyles et les groupements allyles, c'est-à-dire que l'amine primaire de départ de formule générale RNH₂ est une hétéroarylméthylamine ou une allylamine.

Comme exemples de telles amines, on peut citer le 2-méthylaminothiophène, l'allylamine, la 3-méthylaminopyridine, le 2-méthylaminofuranne et la cinnamylamine.

Toutefois, d'autres groupements organiques sont suffisamment électrophiles et conviennent dans le cadre de la présente invention.

Le groupement organique R peut notamment aussi être un groupement propargyle éventuellement substitué, un groupement sulfényle, le groupement triméthylsilyléthyle ou un groupement naphtylméthyle, de préférence le groupement 1-naphtylméthyle.

L'amine de départ peut aussi être une alkylamine ou une amine aliphatique comportant un ou des groupements libérables, par exemple une méthylamine dont le groupement méthyle est substitué par un groupement R'-O-, R'-S- ou R'-N- dans lequel R' désigne un radical organique quelconque, une acylamine, par exemple acétamide ou propylamide, une halogénoamine, par exemple la chloramine, une sulfonamide, une phosphoramide, une silylamine, une nitrosamine.

Selon la présente invention, le dérivé α,β-dicarbonylé est de préférence le glyoxal, quelle que soit sa forme, notamment sous forme libre, hydratée ou polymérisée, ou bien encore un dérivé de l'acide oxalique, par exemple un ester ou un amide.

Le glyoxal est très particulièrement préféré.

La première étape de réaction du dérivé α,β-dicarbonylé avec l'amine primaire permettant de former le dérivé hexasubstitué de l'hexaazaisowurtzitane est généralement réalisée dans un milieu solvant polaire, de préférence un mélange d'un solvant organique polaire et d'eau. Selon une variante préférée, le solvant organique polaire et l'eau sont miscibles dans les proportions utilisées. Le rapport pondéral solvant organique polaire/eau est de préférence compris entre 5 et 20, mieux encore entre 6 et 10, lorsque la totalité des réactifs est présente dans le milieu réactionnel.

Comme exemples de solvant organique polaire utilisable, on peut citer l'acétonitrile, les alcools comme le méthanol, l'éthanol et le propanol, le nitrométhane et le tétrahydrofuranne. L'acétonitrile et le méthanol sont particulièrement préférés, plus particulièrement l'acétonitrile.

Bien que la réaction du dérivé α-β-dicarbonylé avec l'amine primaire puisse se produire, mais à vitesse plus lente, sans catalyseur, on préfère la mettre en oeuvre en présence d'un catalyseur acide, de type acide de Brönsted ou de type acide de Lewis.

Ce catalyseur peut être un acide minéral comme l'acide perchlorique, l'acide sulfurique, l'acide chlorhydrique ou l'acide nitrique, ou bien encore un acide organique comme l'acide formique ou l'acide acétique. L'acide formique est particulièrement préféré.

On peut aussi utiliser un acide de Lewis stable en milieu aqueux tel que notamment un trifluorométhylsulfonate de lanthanide, notamment le trifluorométhylsulfonate d'ytterbium III.

On utilise de préférence entre 25 % et 60 % molaires de catalyseur par rapport au dérivé α,β-dicarbonylé, mieux encore entre 30 % et 50 % molaires.

Par ailleurs, bien que la stoechiométrie théorique de la réaction amine/dérivé α,β-dicarbonylé soit de 2, et qu'un tel rapport puisse être utilisé, on obtient de meilleurs rendements en utilisant un rapport molaire amine primaire/dérivé α,β-dicarbonylé compris entre 2,5 et 3,5, par exemple voisin de 3.

La concentration en amine primaire dans le milieu réactionnel est de préférence comprise entre 1 mole/litre et 6 moles/litre.

Selon une variante particulièrement préférée, on ajoute lentement le dérivé α,β-dicarbonylé dans le milieu réactionnel contenant au préalable la totalité de l'amine, le solvant organique polaire et éventuellement le catalyseur.

La température du milieu réactionnel est de préférence comprise entre 0°C et 25°C, de préférence entre 0°C et 15°C. Mieux encore, elle est tout d'abord comprise entre 0°C et 5°C, puis elle est progressivement augmentée, jusqu'à une valeur comprise entre 15°C et 25°C.

La durée de la réaction peut être variable selon les produits et les conditions opératoires. Elle est en général comprise entre 1h et 20h.

Le dérivé hexasubstitué de l'hexaazaisowurtzitane obtenu est ensuite de préférence isolé du milieu réactionnel, puis purifié selon des moyens usuels tels que filtration, décantation, extraction à l'éther, purification sur gel de silice ou d'alumine.

L'HNIW est ensuite directement obtenu, en une seule étape réactionnelle, par nitration de ce dérivé hexasubstitué de l'hexaazaisowurtzitane, à l'aide d'un réactif nitrant usuel. On peut notamment utiliser, comme réactif nitrant, N₂O₅, le tétrafluoroborate de nitronium, l'acide nitrique concentré, un oléum nitrique, un mélange acétonitrique ou un mélange sulfonitrique. Les mélanges sulfonitriques sont particulièrement préférés, notamment ceux ayant un rapport pondéral HNO3/H₂SO₄ compris entre 0,7 et 4, mieux encore compris entre 1 et 2.

Sans que cela soit nécessaire, on peut réaliser cette étape de nitration en présence d'un solvant organique, notamment un solvant organique halogéné tel que le dichlorométhane ou le chloroforme.

On peut aussi, de façon préférée, réaliser le mélange des réactifs (dérivé hexasubstitué de l'hexaazaisowurtzitane et réactif nitrant) en présence du solvant organique, à une température par exemple comprise entre -10°C et 25°C, éliminer ensuite tout ou partie du solvant, puis poursuivre la réaction de nitration à une température plus élevée, par exemple comprise entre 45°C et 85°C.

De façon générale, selon la présente invention, on utilise un très large excès molaire d'agent nitrant par rapport à la stoechiométrie de la réaction.

L'hydrolyse du milieu réactionnel permet ensuite de faire précipiter l'HNIW formé, que l'on peut alors isoler et récupérer par exemple par simple filtration.

La présente invention a également pour objet les nouveaux dérivés précités intermédiaires hexasubstitués de l'hexaazaisowurtzitane formés, isolés et identifiés, à savoir les dérivés 2,4,6,8,10,12-hexaR-2,4,6,8,10,12-hexaazatétracyclo (5.5.0.0^{5,9}.0^{3,11}) dodécane, pour lesquels R représente un groupement organique électrophile, c'est-à-dire suffisamment électrophile pour que les 6 liaisons N-R soient directement nitrolysables, de préférence un groupement hétéroarylméthyle, un groupement allyle, un groupement propargyle, le groupement triméthylsilyléthyle, un groupement naphtylméthyle ou un groupement sulfényle.

Les exemples non limitatifs suivants illustrent l'invention et les avantages qu'elle procure.

### Exemple 1 : Synthèse du 2,4,6,8,10,12-hexa(thiophène-2 méthyle)-2,4,6,8,10,12-hexaazatétracyclo (5.5.0.0^{5,9}.0^{3,11})dodécane, encore appelé hexa(thiophène-2 méthyle)isowurtzitane.

La formule développée du radical thiophène-2 méthyle est

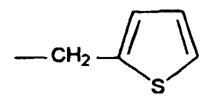

Dans un ballon de 250ml muni d'un thermomètre et refroidi entre 0°C et 10°C par un bain de glace, on introduit successivement 100ml d'acétonitrile, 10ml d'eau distillée, 0,093 mole de 2-méthylaminothiophène et 0,0093 mole d'acide formique. On coule ensuite goutte à goutte (durée 10 min environ) 0,031 mole de glyoxal sous forme d'une solution aqueuse à 40 %.

On agite le milieu réactionnel durant 18h en laissant revenir sa température à la température ambiante (20°C environ).

Après arrêt de l'agitation, une gomme épaisse décante au fond du ballon. On soutire le surnageant et on dissout cette gomme dans 40ml de chloroforme.

Après séchage de la solution chloroformique sur sulfate de sodium puis filtration, on concentre sous vide cette solution. On récupère un solide jaune paille souillé par une huile visqueuse, que l'on purifie sur gel de silice à l'aide d'un mélange éluant hexane/éther diéthylique dans les proportions volumiques respectives 5/1.

Le produit purifié ainsi obtenu est du 2,4,6,8,10,12-hexa(thiophène-2méthyle)-2,4,6,8,10,12-hexaazatétracyclo (5.5.0.0^{5,9}.0^{3,11}) dodécane identifié par analyses spectroscopiques RMN¹H et RMN¹³C dans CDCl₃, ainsi que par rayons X.

Le rendement par rapport au glyoxal en produit purifié obtenu est de 40 %.
RMN¹H (ppm, CDCl₃) :
   - δ =: 3.83,s(2H), CH cage
   4.24, s et 4.35, q(16H), CH cage et CH₂
   6.6-7.3, m, (18H), CH aromatiques
RMN¹³C (ppm, CDCl₃) :
   - δ =: 51.3, 52.8 CH₂
   76.4, 81.6 CH
   125.26, 125.37, 125.43, 126.0, 126.9, 127.0,
   145.6, 146.1 C aromatiques.

La structure du composé a été confirmée par une détermination par rayons X sur monocristal :
- Groupe d'espace :: P21/n
- Paramètres de maille :: a = 12,56799 Å
b = 15,82700 Å
c = 18,92300 Å
α = 90,000°
β = 106,320°
γ = 90,000°

### Exemple 2 : Synthèse du 2,4,6,8,10,12-hexacinnamyle-2,4,6,8,10,12-hexaazatétracyclo(5.5.0.0^{5,9}.0^{3,11}) dodécane, encore appelé hexacinnamyleisowurtzitane

La formule développée du radical cinnamyle est

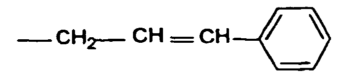

On opère comme selon l'exemple 1, mais en utilisant d'une part de la cinnamylamine au lieu de 2-méthylaminothiophène, et d'autre part un mélange éluant de purification hexane/éther diéthylique dans les proportions volumiques respectives 5/2 au lieu de 5/1.

Le produit purifié ainsi obtenu est du 2,4,6,8,10,12-hexacinnamyle-2,4,6,8,10,12-hexaazatétracyclo(5.5.0.0^{5,9}.0^{3,11}) dodécane identifié par RMN¹H et RMN¹³C. Le rendement par rapport au glyoxal en produit purifie obtenu est de 18 %.
RMN¹H (ppm, CD₃COCD₃) :
   - δ =: 3.98-4.04, m, (12H), CH₂ allyliques
   4.20, s, (2H), CH cage
   4.46, s, (4H), CH cage
   6.33-6.79, m, (12H), CH éthyléniques
   7.3-7.6, m, (30H), CH aromatiques
RMN¹³C (ppm, CD₃COCD₃) :
   - δ =: 55.7, 56.4 CH₂ allyliques
   78.2, 82.2 CH cage
   127.48, 127.55, 128.27, 128.33, 129.65, 129.77,
   130.45, 130.74, 132.43, 132.57, 138.7 138.8 C
   aromatiques et CH éthyléniques.

### Exemple 3 : Synthèse du 2,4,6,8,10,12-hexa (pyridyl-3-méthyle)-2,4,6,8,10,12-hexaazatétracyclo (5.5.0.0^{5,9}.0^{3,11}) dodécane, encore appelé hexa (pyridyl-3 méthyle) isowurtzitane

La formule développée du radical pyridyl-3 méthyle est

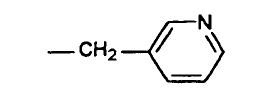

Dans un ballon de 250ml muni d'un thermomètre et refroidi entre 0°C et 10°C par un bain de glace, on introduit successivement 100ml d'acétonitrile, 10ml d'eau distillée, 0,093 mole de 3-méthylaminopyridine et 0,0093 mole d'acide formique. On coule ensuite goutte à goutte (durée 10min environ) 0,031 mole de glyoxal sous forme d'une solution aqueuse à 40 %.

On agite le milieu réactionnel durant 18h en laissant revenir sa température à la température ambiante (20°C environ), puis on ajoute 150ml d'éther diéthylique au milieu réactionnel.

Après décantation, on récupère la phase organique que l'on sèche sur sulfate de sodium, puis que l'on filtre et concentre sous vide. Le produit brut ainsi obtenu est purifié sur alumine basique désactivée avec 6 % d'eau en éluant d'abord avec un mélange chloroforme/éther diéthylique/triéthylamine dans les proportions volumiques respectives 5/2/0,1, puis avec un mélange chloroforme/triéthylamine dans les proportions volumiques respectives 5/0,1.

Le produit purifié ainsi obtenu est du 2,4,6,8,10,12-hexa(pyridyl-3 méthyle)-2,4,6,8,10,12-hexaazatétracyclo(5.5.0.0^{5,9}.0^{3,11}) dodécane identifié par RMN¹H et RMN¹³C.

Le rendement en produit purifié obtenu par rapport au glyoxal est de 19 %.
RMN¹H (ppm,CD₃COCD₃) :
   - δ =: 3.89,s, (2H), CH cage
   4.24-4.58, m, (16H), CH cage et CH₂
   7.30-8.60, m, (24H), CH aromatiques
RMN¹³C (ppm, CD₃COCD₃) :
   - δ =: 54.3, 55.1 CH₂
   77.1, 81.8 CH cage
   124.1, 135.4, 135.6, 136.4, 137.1, 149.3,
   150.2, 151.1 CH aromatiques.

### Exemple 4 : Synthèse du 2,4,6,8,10,12-hexaallyle-2,4,6,8,10,12-hexaazatétracyclo(5.5.0.0^{5,9}.0^{3,11}) dodécane, encore appelé hexaallylisowurtzitane

Dans un ballon de 250ml muni d'un thermomètre et refroidi entre 0°C et 2°C par un bain de glace, on introduit successivement 150ml d'acétonitrile, 0,93 mole d'allylamine et 0,13 mole d'acide formique dans 1g d'eau.

On coule ensuite goutte à goutte (durée 75min environ) 0,31 mole de glyoxal sous forme d'une solution aqueuse à 40 %.

On agite le milieu réactionnel pendant 45min à 0°C puis on le filtre sous atmosphère d'argon, ce que permet d'obtenir directement, sans étape de purification, du 2,4,6,8,10,12-hexaallyle-2,4,6,8,10,12-hexaazatétracyclo (5.5.0.0^{5,9}.0^{3,11}) dodécane pur, identifié par RMN¹H et RMN¹³C, avec un rendement de 20 % par rapport au glyoxal.
RMN¹H (ppm, CDCl₃) :
   - δ =: 3.55-3.70, m, (12H), CH₂ allyliques
   3.85, s, (2H), CH cage
   4.16, s, (4H), CH cage
   5.0-5.3, m, (12H), CH₂ éthyléniques
   5.75-6.0, m, (6H), CH éthyléniques
RMN¹³C (ppm, CDCl₃) :
   - δ =: 56.4, 56.7 CH₂ allyliques
   77.7, 80.8 CH cage
   116.6, 117.6, CH₂ éthyléniques
   137.9, 138.4 CH éthyléniques.

### Exemple 5 : Synthèse du 2,4,6,8,10,12-hexafurfurylméthyle-2,4,6,8,10,12-hexaazatétracyclo (5.5.0.0^{5,9}.0^{3,11}) dodécane, encore appelé hexafurfurylméthylisowurtzitane

La formule développée du radical furfurylméthyle, encore appelé furyl-2 méthyle, est

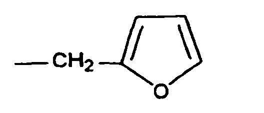

Dans ballon de 500ml muni d'un thermomètre et refroidi à 0°C par un bain de glace, on introduit successivement 150ml d'acétonitrile, 0,93 mole de 2-méthylaminofuranne et 0,13 mole d'acide formique dans 1g d'eau.

On coule ensuite goutte à goutte (durée 20min environ) 0,31 mole de glyoxal sous forme d'une solution aqueuse à 40 %, en maintenant la température entre 2°C et 5°C. On augmente ensuite progressivement, en 1h, la température jusqu'à 10°C, puis en 2h progressivement jusqu'à 15°C. On agite alors le milieu réactionnel durant 2h à 15°C.

Après décantation, on recueille et concentre la phase lourde, puis on reprend le résidu obtenu par 11 d'éther diéthylique.

Après lavage à l'eau (3 fois 60ml) de cette phase éthérée, puis séchage sur sulfate de magnésium et filtration, on chasse l'éther pour finalement recueillir du 2,9,6,8,10,12-hexafurfurylméthyle-2,4,6,8,10,12-hexaazatétracyclo(5.5.0.0^{5,9}.0^{3,11}) dodécane, identifié par RMN¹H et RMN¹³C, avec un rendement de 60 % par rapport au glyoxal.
RMN¹H (ppm, CDCl₃) :
   - δ =: 3.58, s, (2H), CH cage
   4.05, s, (4H), CH₂ et 4.09, q, (8H) CH₂
   4.23, s, (4H), CH cage
   5.93-6.40, m, (12H), CH aromatiques
   7.35, m, (6H), CH aromatiques
RMN¹³C (ppm, CDCl₃) :
   - δ =: 49.2, 50.3 CH₂
   77.2, 80.1 CH cage
   108.1, 108.4, 110.7, 142.4, 142.5, 154.0, 154.8
   CH aromatiques

### Exemple 6 : Synthèse du 2,4,6,8,10,12-hexapropargyl-2,4,6,8,10,12-hexaazatétracyclo(5.5.0.0^{5,9}.0^{3,11})dodécane, encore appelé hexapropargylhexaazaisowurtzitane.

Dans un ballon bicol de 250ml, refroidi à 0-2°C, on introduit 100ml d'acétonitrile, 10ml d'eau, 0,0093mole d'acide formique et 0,093 mole de propargylamine.

On coule ensuite goutte à goutte 0,031 mode de glyoxal sous forme d'une solution aqueuse à 40 %.

On agite le milieu réactionnel pendant 75min entre 0 et 2°C puis on le concentre sous pression réduite.

Après extraction par le dichlorométhane, puis séchage sur sulfate de sodium, on évapore le solvant d'extraction, ce qui permet d'obtenir un produit brut que l'on purifie sur gel d'alumine basique. On obtient finalement 2,11g d'un solide blanc, identifié par RMN¹H, RMN¹³C, par son point de fusion, analyse élémentaire et radiocristallographie de rayons X, comme étant de l'hexapropargylhexaazaisowurtzitane.

Le rendement par rapport au glyoxal en produit purifié est de 17 %.
Point de fusion : 114,0°C-114,3°C
Analyse élémentaire :
   C : 71,7 % (théorie 72,7 %)
   H : 6,2 % (théorie 6,1 %)
   N : 19,8 % (théorie 21,2 %)
RMN¹H (ppm, CDCl₃) :
   - δ =: 2.21 (t, 4H, J = 2Hz)
   2.28 (t, 2H, J = 2Hz)
   3.78 (m, 12H)
   4.15 (s, 2H)
   4.47 (s, 4H)
RMN¹³C (ppm, CDCl₃) :
   - δ =: 41.1 (CH₂)
   42.1 (CH₂)
   71.2, 73.0 (Q)
   75.5, 80.7, 80,9, 81,5 (CH).

L'analyse par radiocristallographie de rayons X à partir d'un monocristal obtenu par évaporation d'une solution du produit dans un mélange méthanol/éthanol confirme la structure du produit.

### Exemple 7 : Synthèse du 2,4,6,8,10,12-hexa(parachlorobenzènesylfényl)-2,4,6,8,10,12-hexaazatétracyclo (5.5.0.0^{5,9}.0^{3,11})dodécane, encore appelé hexaparachlorobenzènesulfénylhexaazaisowurtzitane

On opère comme selon l'exemple 6, excepté que l'amine employée est la parachlorobenzènesulfénamide (au lieu de propargylamine) et que l'on n'introduit pas d'eau ni de catalyseur acide formique. La structure du produit obtenu a été caractérisée en DEPT et RMN¹³C, grâce notamment aux CH (cage) résonnant à 88,3 et 88,8ppm.

### Exemples 8 et 9 : Synthèse du 2,4,6,8,10,12-hexa (1-naphtylméthyl)-2,4,6,8,10,12-hexaazatétracyclo (5.5.0.0^{5,9}.0^{3,11})dodécane, encore appelé hexa-1-naphtylméthylhexaazaisowurtzitane

### Exemple 8 :

On opère comme selon l'exemple 6, excepté que l'amine employée est la 1-naphtylméthylamine (au lieu de propargylamine) et qu'on laisse la réaction se produire 36h à la température ambiante (20°C environ).

Le rendement par rapport au glyoxal en produit recherché purifié est de 25 %. Son point de fusion est 244-245°C.

Le produit a été identifié comme étant de l'hexa-1-naphtylméthylhexaazaisowurtzitane par RMN¹H et RMN¹³C:
RMN¹H (ppm, CDCl₃) :
   - δ =: 7.3 - 8.2 (m, 34H)
   6.89 (t, 4H)
   5.90 (d, 4H)
   4.69 (s, 4H)
   4.60 (s, 4H)
   4.33 (AB, 8H)
   3.45 (s, 2H)
RMN¹³C (ppm, CDCl₃) :
   - δ =: 78.8 (CH)
   78.0 (CH)
   55.5 (CH₂)
   53.5 (CH₂).

### Exemple 9 :

On opère comme selon l'exemple 8, excepté qu'on remplace l'acide formique par le trifluorométhylsulfonate d'ytterbium III.

Le rendement par rapport au glyoxal en hexa-1-naphtylméthylhexaazaisowurtzitane purifié obtenu est de 62 %.

### Exemple 10 : Synthèse de l'hexanitrohexaazaisowurtzitane par nitration directe de l'hexafurfurylméthylisowurtzitane

Dans un ballon de 100ml équipé d'une agitation mécanique, d'un réfrigérant, d'une sonde de température et d'une ampoule de coulée, on introduit, sous atmosphère d'argon, 12g d'acide nitrique à 100 %. A l'aide d'un bain de glace-sel, on porte la température à 10°C, puis on ajoute 8g d'acide sulfurique concentré, puis 10ml de dichlorométhane sec.

On ajoute ensuite goutte à goutte (durée 20min environ), à une température comprise entre -5°C et -3°C, une solution de 0,0014 mole d'hexafurfurylméthylisowurtzitane obtenu selon l'exemple 5 dans 5ml de dichlorométhane.

On enlève alors le bain de glace-sel afin de laisser la température remonter jusqu'à 10°C.

On chasse ensuite le dichlorométhane, sous balayage d'argon, en portant la température du milieu réactionnel à 30°C.

On élève ensuite progressivement, en 4h, la température du milieu réactionnel jusqu'à 65°C.

Après hydrolyse dans la glace, puis filtration, on récupère 71,5mg (rendement 12 %) d'un solide pur, identifié comme étant de l'hexanitrohexaazaisowurtzitane par spectrométries RMN¹H, RMN¹³C et IRFT, ainsi que par chromatographe liquide (HPLC) par comparaison du temps de rétention avec un échantillon authentique.

Le spectre Infra-Rouge par Transformée de Fourier (IRTF) est celui de la forme polymorphe gamma.

### Exemple 11 : Synthèse de l'hexanitrohexaazaisowurtzitane par nitration directe de l'hexaallylisowurtzitane par le tétrafluoroborate de nitronium

A une température de 5°C, on introduit 0,0147 mole de NO₂BF₄ dans une solution de 0,00254 mole d'hexaallyisowurtzitane synthétisé selon l'exemple 4 dans 25ml de dichlorométhane.

On laisse ensuite le milieu se réchauffer jusqu'à la température ambiante (20°C environ).

La présence d'hexanitrohexaazaisowurtzitane est détectée dans le milieu réactionnel par HPLC.

### Exemple 12 : Synthèse de l'hexanitrohexaazaisowurtzitane par nitration directe de l'hexaallylisowurtzitane par un mélange sulfonitrique

On introduit 0,00245 mole d'hexallylisowurtzitane synthétisé selon l'exemple 4 dans un mélange, à -5°C, de 0,171 mole d'acide nitrique et 0,03425 mole d'acide sulfurique. On chauffe ensuite lentement le milieu réactionnel jusqu'à 70°C, en 5 heures. Il se forme un solide jaune, isolé par filtration du milieu, contenant de l'hexanitrohexaazaisowurtzitane identifié par RMN¹H et HPLC.

## Revendications

1. Procédé de synthèse de l'hexanitrohexaazaisowurtzitane comprenant une première étape de réaction d'un dérivé α,β-dicarbonylé avec une amine primaire permettant de former un dérivé hexasubstitué de l'hexaazaisowurtzitane, **caractérisé en ce que** l'hexanitrohexaazaisowurtzitane est directement obtenu, en une seule étape réactionnelle, par nitration du dérivé hexasubstitué de l'hexaazaisowurtzitane précité.

2. Procédé de synthèse selon la revendication 1, **caractérisé en ce que** le dérivé α,β-dicarbonylé est le glyoxal.

3. Procédé de synthèse selon la revendication 1 ou 2, **caractérisé en ce que** l'amine primaire répond à la formule générale RNH₂ dans laquelle le groupement R est un groupement organique électrophile.

4. Procédé selon la revendication 3, **caractérisé en ce que** l'amine primaire est choisi dans le groupe constitué par les hétéroarylméthylamines et les allylamines.

5. Procédé selon la revendication 4, **caractérisé en ce que** l'amine primaire est l'allylamine ou le 2-méthylaminofuranne.

6. Procédé selon l'une quelconque des revendication 1 à 5, **caractérisé en ce que** la première étape de réaction d'un dérivé α,β-dicarbonylé avec une amine primaire est réalisée dans un mélange d'un solvant organique polaire et d'eau, en présence d'un catalyseur acide.

7. Procédé selon la revendication 6, **caractérisé en ce que** le rapport molaire catalyseur acide/dérivé α,β-dicarbonylé est compris entre 0,25 et 0,6.

8. Procédé selon l'une quelconque des revendications 1 à 7, **caractérisé en ce que** le rapport molaire amine primaire/ dérivé α,β-dicarbonylé est compris entre 2,5 et 3,5.

9. Procédé selon l'une quelconque des revendications 1 à 8, **caractérisé en ce que** la température de la réaction de l'amine primaire avec le dérivé α,β-dicarbonylé est comprise entre 0°C et 25°C.

10. Procédé selon l'une quelconque des revendications 1 à 9, **caractérisé en ce que** le dérivé hexasubstitué de l'hexaazaisowurtzitane formé est isolé du milieu réactionnel avant nitration.

11. Procédé selon l'une quelconque des revendications 1 à 10, **caractérisé en ce que** la nitration du dérivé hexasubstitué de l'hexaazaisowurtzitane est réalisée à l'aide d'un mélange sulfonitrique.

12. Procédé selon l'une quelconque des revendications 1 à 11, **caractérisé en ce que** la nitration du dérivé hexasubstitué de l'hexaazaisowurtzitane est réalisée en présence d'un solvant organique.

13. Procédé selon la revendication 12, **caractérisé en ce que**, pour réaliser l'étape de nitration du dérivé hexasubstitué de l'hexaazaisowurtzitane, on mélange les réactifs en présence du solvant organique à une température comprise entre -10°C et 25°C, on élimine ensuite tout ou partie du solvant organique, puis on poursuit la réaction de nitration à une température comprise entre 45°C et 85°C.

14. Dérivés 2,4,6,8,10,12-hexa R-2,4,6,8,10,12-hexaazatétracyclo(5.5.0.0^{5.9}.0^{3.11}) dodécane, **caractérisés en ce que** le groupement R est choisi dans le groupe constitué par les groupements hétéroarylméthyles, les groupements allyles, les groupements propargyles, le groupement triméthyl-silyléthyle, les groupements naphtylméthyles et les groupements sulfényles.

## Claims

1. Process for the synthesis of hexanitrohexaazaisowurtzitane comprising a first stage of reaction of an α,β-dicarbonyl derivative with a primary amine which makes it possible to form a hexasubstituted hexaazaisowurtzitane derivative, **characterized in that** the hexanitrohexaazaisowurtzitane is obtained directly, in a single reaction stage, by nitration of the above-mentioned hexasubstituted hexaazaisowurtzitane derivative.

2. The synthetic process according to claim 1, **characterized in that** the α,β-dicarbonyl derivative is glyoxal.

3. The synthetic process according to claim 1 or 2, **characterized in that** the primary amine corresponds to the general formula RNH₂ in which the R group is an electrophilic organic group.

4. The process according to claim 3, **characterized in that** the primary amine is chosen from the group consisting of heteroarylmethylamines and allylamines.

5. The process according to claim 4, **characterized in that** the primary amine is allylamine or 2-methylaminofuran.

6. The process according to any one of claims 1 to 5, **characterized in that** the first stage of reaction of an α, β -dicarbonyl derivative with a primary amine is carried out in a mixture of a polar organic solvent and of water, in the presence of an acid catalyst.

7. The process according to claim 6, **characterized in that** the acid catalyst/ α, β-dicarbonyl derivative molar ratio is between 0.25 and 0.6.

8. The process according to any one of claims 1 to 7, **characterized in that** the primary amine/ α, β -dicarbonyl derivative molar ratio is between 2.5 and 3.5.

9. The process according to any one of claims 1 to 8, **characterized in that** the temperature of the reaction of the primary amine with the α, β-dicarbonyl derivative is between 0°C and 25°C.

10. The process according to any one of claims 1 to 9, **characterized in that** the hexasubstituted hexaazaisowurtzitane derivative formed is isolated from the reaction medium before nitration.

11. The process according to any one of claims 1 to 10, **characterized in that** the nitration of the hexasubstituted hexaazaisowurtzitane derivative is carried out using a sulphuric acid/nitric acid mixture.

12. The process according to any one of claims 1 to 11, **characterized in that** the nitration of the hexasubstituted hexaazaisowurtzitane derivative is carried out in the presence of an organic solvent.

13. The process according to claim 12, **characterized in that**, in order to carry out the stage of nitration of the hexasubstituted hexaazaisowurtzitane derivative, the reactants are mixed in the presence of the organic solvent at a temperature of between -10°C and 25°C, all or part of the organic solvent is subsequently removed and then the nitration reaction is continued at a temperature of between 45°C and 85°C.

14. 2 , 4 , 6 , 8, 10 , 12 - hexa R - 2, 4 , 6 , 8, 10 , 12 - hexaazatetracyclo(5.5.0.0^{5,9}.0^{3,11}) dodecane derivatives, wherein R is chosen from the group consisting of heteroarylmethyl groups, allyl groups, propargyl groups, trimethylsilylethyl groups, naphtylmethyl groups and sulphenyl groups.

## Patentansprüche

1. Verfahren zur Herstellung von Hexanitrohexaazaisowurtzitan umfassend eine erste Reaktionsstufe eines α, β-Dicarbonylderivats mit einem primären Amin, wobei ein hexasubstituiertes Derivat des Hexaazaisowurtzitans gebildet wird, **dadurch gekennzeichnet, dass** das Hexanitrohexaazaisowurtzitan direkt in einer einzigen Reaktionsstufe durch Nitrierung des vorstehend erwähnten hexasubstituierten Derivats des Hexaazaisowurtzitan erhalten wird.

2. Verfahren zur Herstellung nach Anspruch 1, **dadurch gekennzeichnet, dass** das α,β-Dicarbonylderivat Glyoxal ist.

3. Verfahren zur Herstellung gemäß Anspruch 1 oder 2, **dadurch gekennzeichnet, dass** das primäre Amin der allgemeinen Formel RNH₂ entspricht, in der die Gruppe R eine organische, elektrophile Gruppe ist.

4. Verfahren nach Anspruch 3, **dadurch gekennzeichnet, dass** das primäre Amin ausgewählt ist aus der Gruppe der Heteroarylmethylamine und der Allylamine.

5. Verfahren nach Anspruch 4, **dadurch gekennzeichnet, dass** das primäre Amin Allylamin oder 2-Methylaminofuran ist.

6. Verfahren nach mindestens einem der Ansprüche 1-5, **dadurch gekennzeichnet, dass** die erste Reaktionstufe eines α, β-Dicarbonylderivats mit einem primären Amin in einem Gemisch eines organischen, polaren Lösungsmittel und Wasser in Gegenwart eines sauren Katalysators durchgeführt wird.

7. Verfahren nach Anspruch 6, **dadurch gekennzeichnet, dass** das molare Verhältnis saurer Katalysator/ α, β-Dicarbonylderivat zwischen 0,25 und 0,6 liegt.

8. Verfahren nach mindestens einem der Ansprüche 1-7, **dadurch gekennzeichnet, dass** das molare Verhältnis primäres Amin/ α, β-Dicarbonylderivat zwischen 2,5 und 3,5 liegt.

9. Verfahren nach mindestens einen der Ansprüche 1-8, **dadurch gekennzeichnet, dass** die Temperatur der Reaktion des primären Amins mit dem α, β-Dicarbonylderivat zwischen 0°C und 25°C liegt.

10. Verfahren nach mindestens einem der Ansprüche 1-9, **dadurch gekennzeichnet, dass** das gebildete hexasubstituierte Derivat des Hexaazaisowurtzitans aus dem Reaktionsmilieu vor der Nitrierung isoliert wird.

11. Verfahren nach mindestens einem der Ansprüche 1-10, **dadurch gekennzeichnet, dass** die Nitrierung des hexasubstituierten Derivats des Hexaazaisowurtzitans mit Hilfe eines Sulfosalpetergemisches durchgeführt wird.

12. Verfahren nach mindestens einem der Ansprüche 1-11, **dadurch gekennzeichnet, dass** die Nitrierung des hexasubstituierten Derivats des Hexaazaisowurtzitans in Gegenwart eines organischen Lösungsmittels durchgeführt wird.

13. Verfahren nach Anspruch 12, **dadurch gekennzeichnet, dass** man, um die Stufe der Nitrierung des hexasubstituierten Dervats des Hexaazaisowurtzitans durchzuführen, die Reaktionsteilnehmer in Gegenwart eines organischen Lösungsmittel bei einer Temperatur zwischen -10°C und 25°C mischt, anschließend das Ganze oder einen Teil des organischen Lösungsmittels entfernt, dann die Nitrierungsreaktion bei einer Temperatur zwischen 45°C und 85°C fortsetzt.

14. 2, 4, 6, 8, 10, 12-Hexa R-2, 4, 6, 8, 10, 12-hexaazatetracyclo(5.5.0.0^{5,9}.0^{3.11})dodecanderivate, **dadurch gekennzeichnet, dass** die Gruppe R ausgewählt ist aus der Gruppe, welche besteht aus Heteroarylmethylgruppen, Allylgruppen, Propargylgruppen, Trimethylsilylethylgruppen, Naphtylmethylgruppen und Sulfenylgruppen.
